(19) Europäisches Patentamt
European Patent Office
Office européen des brevets

(11) EP 0 461 392 B1

(12) **EUROPÄISCHE PATENTSCHRIFT**

(45) Veröffentlichungstag und Bekanntmachung des Hinweises auf die Patenterteilung:
**10.01.1996 Patentblatt 1996/02**

(51) Int Cl.$^6$: **G01N 33/52**, G01N 31/22
// G01N33/84

(21) Anmeldenummer: **91107495.3**

(22) Anmeldetag: **08.05.1991**

(54) **Testträger zur Bestimmung von Ionen**

Test carrier for the determination of ions

Support d'essai pour la détermination d'ions

(84) Benannte Vertragsstaaten:
**AT BE CH DE DK ES FR GB GR IT LI LU NL SE**

(30) Priorität: **15.05.1990 DE 4015590**

(43) Veröffentlichungstag der Anmeldung:
**18.12.1991 Patentblatt 1991/51**

(73) Patentinhaber:
**BOEHRINGER MANNHEIM GMBH**
**D-68298 Mannheim (DE)**

(72) Erfinder:
- **Vogel, Peter, Dr. rer. nat.**
  **W-6944 Hemsbach (DE)**
- **Thym, Detlef, Dr. rer. nat.**
  **W-6800 Mannheim 1 (DE)**
- **Fritz, Michael**
  **W-6843 Biblis (DE)**
- **Mosoiu, Dan**
  **W-6703 Limburgerhof (DE)**

(56) Entgegenhaltungen:
**EP-A- 0 016 387**
**EP-A- 0 125 554**
**EP-A- 0 153 641**
**EP-A- 0 175 990**
**EP-A- 0 207 392**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 461 392 B1

Printed by Jouve, 75001 PARIS (FR)

## Beschreibung

Die Erfindung betrifft einen Testträger zur Bestimmung von Ionen insbesondere in wässrigen Flüssigkeiten und besonders bevorzugt in Körperflüssigkeiten, wie Blut, Plasma, Serum oder Urin.

Die Bestimmung von Ionen, speziell Alkali- und Erdalkali-Ionen in Flüssigkeiten ist von großer Bedeutung. Erinnert sei hier nur an die Bestimmung der Wasserhärte, die Analyse von Abwässern und dergleichen.

Die Bestimmung von Alkali- und Erdalkali-Ionen speziell in Körperflüssigkeiten spielt eine hervorragende Rolle in der medizinischen Diagnostik. So werden Kalium- und Natrium-Bestimmungen im Klinik- und Notfall-Labor für die Diagnose und Verlaufskontrolle von Herz-Kreislauf-Erkrankungen, Erkrankungen der Muskulatur, Nierenerkrankungen, Schockzuständen verschiedener Genese und dergleichen benötigt. Die Bestimmung von Calcium ist wichtig für die Diagnose von Knochen-Erkrankungen. Lithium-Bestimmungen werden für die Therapiekontrolle lithiumhaltiger Antidepressiva benötigt.

Ionen-Bestimmungen, insbesondere im klinischen Labor werden überwiegend mit Hilfe der Flammenphotometrie und mittels ionensensitiver Elektroden durchgeführt.

Daneben sind zahlreiche Verfahren bekannt, die die photometrische Analyse von Ionen ermöglichen, d. h. bei denen die Analysereaktion zu einer Farbänderung führt. Ein Überblick über solche Verfahren ist dem Artikel von M. Kolthoff "Application of macrocyclic compounds in chemical analysis" in Analytical Chemistry, 1979, 1R-22R, insbesondere Seite 16R-18R, und den dort zitierten Literaturstellen zu entnehmen.

Diese Verfahren beruhen auf der sogenannten Extraktionsanalyse. Dabei wird ein für die zu bestimmende Ionenart selektiver Komplexbildner (Ionophor) in einer organischen, mit Wasser nicht mischbaren (hydrophoben) Flüssigkeit gelöst. Dieses Testsystem wird mit der wässrigen Probenflüssigkeit (z. B. Blutserum) in intensiven Kontakt gebracht (meist wird heftig geschüttelt). Dabei werden die Ionen komplexiert und treten damit in die organische Phase über.

Für den Farbnachweis dieses Vorgangs sind verschiedene Verfahren bekannt, so insbesondere die Verwendung einer gemeinsam mit den Analytionen in die organische Phase übertretenden Gegenion-Farbsubstanz, die Verwendung eines mit dem Ionophor kovalent gekoppelten Farbstoffes (insgesamt auch als Chromoionophor bezeichnet) oder die Verwendung eines in der organischen Phase befindlichen pH-Indikators, der beim Eintritt des Analytions in die mit Wasser nicht mischbare Flüssigkeit zum Ladungsausgleich ein Proton in die wässrige Probenflüssigkeit abgibt und dadurch seine Farbe ändert. Letzteres Verfahren wird als heterogene pH-Reaktion bezeichnet.

In jüngerer Zeit hat die Analyse mit Hilfe sogenannter Testträger, die auch als Schnelldiagnostica und in der angelsächsischen Literatur als "analysis test carriers" oder "solid state analysis devices" bezeichnet werden, zunehmend an Bedeutung gewonnen. Es sind dies analytische Zubereitungen, bei denen sämtliche für eine Analyse erforderlichen Reagenzien auf oder in saugfähigen oder quellbaren Trägern oder Filmen vorliegen und die in Form einfach zu benutzender Vorrichtungen, sogenannter Testträger, wie Teststreifen oder Slides montiert sind. Nach Befeuchtung mit der Probe, wie einer Körperflüssigkeit läuft die Nachweisreaktion ab. Eine gebildete Farbe als Maß für die Menge des zu bestimmenden Analyts kann dann mit Hilfe einer Farbvergleichstafel oder mit einfachen Reflektionsphotometern ausgewertet werden.

Schnelldiagnostica zeichnen sich insbesondere durch einfache Handhabung aus. Deswegen werden auch bei Benutzung durch wenig trainiertes Laborpersonal oder sogar durch den Patienten Analyseresultate von höchster Genauigkeit erzielt.

In Anbetracht dieser Vorteile hat es nicht an Versuchen gefehlt, auch für die Analyse von Ionen Schnelldiagnostica zu entwickeln, wobei auch hier die aus der klassischen Reagenzglas-Analytik bekannten Ansätze benutzt wurden.

Testträger, die auf den Prinzipien ionenselektiver Elektroden beruhen, sind beispielsweise aus dem US-Patent 4,171,246 bekannt. Sie sind jedoch verhältnismäßig aufwendig aufgebaut und ihre Handhabung wird insbesondere dadurch erschwert, daß zusätzlich zur Probenflüssigkeit eine Referenz flüssigkeit korrekt auf die entsprechende Position des Testträgers aufgegeben werden muß.

Die Übertragung der Extraktionsanalyse auf Testträger ist in dem US-Patent 3,635,679 am Beispiel von zweiwertigen Metallionen beschrieben. Dabei wird eine Filmschicht (Analysefilm) auf Basis eines hydrophoben Polymers (insbesondere Polyvinylchlorid) verwendet, die als zusätzliche Filmbildungskomponente eine hydrophobe organische Flüssigkeit enthält, die üblicherweise als Weichmacher für Kunststoffolien eingesetzt wird. Die EP-B-0 041 175 befaßt sich mit der Anwendung der gleichen Reaktionsprinzipien bei Alkalimetallen.

Die Verwendung eines Analysefilms mit den genannten Filmbildungskomponenten ermöglicht prinzipiell die Analyse von Ionen mit Hilfe eines Schnelldiagnosticums. In der Praxis ergeben sich jedoch große Schwierigkeiten, die überwiegend auf den langsamen Austausch zwischen der wässrigen Probenflüssigkeit und dem hydrophoben Analysefilm zurückzuführen sein dürften. Insbesondere ist die Farbbildung nicht ausreichend intensiv, so daß nur ein verhältnismäßig geringer Signalhub und infolgedessen eine verhältnismäßig schlechte Präzision der Analyse erreicht wird. Außerdem wird nicht im gewünschten Umfang ein definierter Farbumschlag mit einem konstanten Endwert beobachtet, wie dies für eine Analyse mit einer Endpunktbestimmung wünschenswert wäre.

Es sind deshalb bereits zahlreiche spezielle Testschichtstrukturen vorgeschlagen worden, die insoweit Verbesserungen erreichen sollen.

In EP-A-0 125 555 ist eine Ausführungsform beschrieben, bei der die organische Phase als weichgemachter, unpolarer, nicht-poröser Kunststoff-Film vorliegt, in dem Ionophor und Indikator gelöst sind. Wie aus den Beispielen ersichtlich ist, kommt die Nachweisreaktion nicht zum Stillstand, so daß die Bestimmung der Ionen nur durch kinetische Messungen möglich ist. Dies ist insbesondere dann von Nachteil, wenn, wie bei Schnelldiagnostica üblich, ohne die Mitführung eines Standards gemessen werden soll. Kinetische Messungen sind dann sehr anfällig gegen Alterungen der diagnostischen Mittel. Außerdem sind in den Beispielen lediglich Teste für wässrige Lösungen mit oberen Konzentrationsgrenzen bis 3 mmol Kalium/l offenbart, was für die Analyse von Serum diagnostisch unzureichend ist und eine Verdünnung der Probe erfordert. Serum Gesunder weist Kalium-Konzentrationen von etwa 3,5 bis 5,5 mmol/l auf.

In EP-A-0 125 554 ist eine Anwendungsform beschrieben, bei der die organische hydrophobe Phase in Form kleiner Tröpfchen in einer hydrophilen Matrix eingebettet ist. Die offenbarten Reaktionszeiten von mehr als zwei Minuten sind aber für ein Schnelldiagnosticum relativ lang. Außerdem sind solche Emulsionen erfahrungsgemäß schwierig reproduzierbar herzustellen und insbesondere auch über längere Zeiträume in getrocknetem Zustand zu halten. Auch hier bedarf es gemäß den Beispielen für die Analyse von Kalium einer Vorverdünnung.

EP-A-0 141 647 beschreibt eine Testvorrichtung insbesondere zur Bestimmung eines ionischen Analyten in einer Flüssigkeit, enthaltend reflektierende Partikel ohne Hohlraumvolumen, die durch ein polymeres Bindemittel zusammengehalten werden. Auf der Oberfläche der Partikel befindet sich ein Extraktionsmittel für den Analyt und ein Detektionsagens, beispielsweise ein Chromoionophor. Das polymere Bindemittel selbst muß durchlässig für den Analyt bzw. Reaktionsprodukte des Analyts sein. Wie in den Beispielen gezeigt wird, benötigen Testvorrichtungen dieser Art Reaktionszeiten von etwa fünf Minuten. Solche Zeiten sind für ein Schnelldiagnosticum zu lang.

In EP-A-0 153 641 ist beschrieben, daß eine poröse Trägermatrix mit einer organischen, hydrophoben Phase, enthaltend Chromophor und Ionophor imprägniert wird. Papier wird als bevorzugte Trägermatrix beschrieben. Erfahrungsgemäß sind Papiere jedoch relativ ungleichmäßig beschaffen, was dazu führt, daß eine insbesondere für die Kaliumbestimmung notwendige Präzision nicht immer erreicht werden kann. So wird in der genannten Anmeldung erwähnt, daß Variationskoeffizienten bis 4 % erreicht werden.

In EP-A-0 175 990 sind hydrophile, vorzugsweise wasserlösliche Partikel aus entsprechenden organischen Polymeren in eine organische Phase aus hydrophobem, filmbildendem, wasserunlöslichem Polymer eingebettet.

Der Erfindung liegt die Aufgabe zugrunde, einen Schnelltest für die Bestimmung von Ionen zur Verfügung zu stellen, der sich durch eine schnelle Reaktion und intensive Farbbildung und demgemäß durch eine erhöhte Präzision der Analyse auszeichnet.

Diese Aufgabe wird durch die in den Patentansprüchen charakterisierte Erfindung gelöst.

Gegenstand der Erfindung ist ein Testträger zur Bestimmung von Ionen. Er enthält eine Testschicht, die eine flüssigkeitsfeste organische Phase aufweist, die ihrerseits ein hydrophobes Polymer in homogener Mischung mit einer schwer flüchtigen, mit Wasser nicht mischbaren Flüssigkeit enthält. Weiter enthält der Testträger einen Ionophor und ein Chromogen, das in Anwesenheit des zu bestimmenden Ions seine Farbe ändert. Der erfindungsgemäße Testträger ist dadurch gekennzeichnet, daß die Testschicht Partikel mit einer ölzahl von 80 - 200, vorzugsweise 100 - 170 enthält und der Ionophor in der hydrophoben organischen Phase homogen verteilt ist.

Die Testschicht des erfindungsgemäßen Testträgers besitzt eine hochporöse Struktur. Da im Gegensatz zu EP-A-0 153 641 keine Trägermatrix erforderlich ist, entfallen die durch die Struktur des Matrixmaterials hervorgerufenen Probleme bei der optischen Auswertung. Außerdem läßt sich die erfindungsgemäße Testschicht relativ leicht herstellen, was ökonomische Vorteile zur Folge hat.

Durch Verwendung der erfindungsgemäßen Testschicht ist nur eine sehr kurze Reaktionszeit erforderlich. Die Farbbildung ist bereits nach etwa 60 Sekunden abgeschlossen, so daß eine Endpunktbestimmung unter praktischen Gesichtspunkten gut durchführbar ist.

Testschichten, die in einem polymeren Material eingelagerte organische oder anorganische Partikel enthalten, sind bekannt. So wird beispielsweise die Verwendung verschiedener Partikel zur Erzeugung einer porösen Ausbreitungsschicht in der US-Patentschrift 3,992,158 beschrieben. Die EP-B-0 016 387 richtet sich auf eine Analyseschicht, bei der anorganische oder organische Partikel in einem bestimmten Größen- und Konzentrationsbereich als Filmöffner eingesetzt werden. Die EP-B-0 013 156 beschreibt eine Teilchenverbundstruktur, bei der organische Polymerteilchen mit Hilfe einer geringen Menge an Bindemittel zu einer offenporigen Verbundstruktur verbunden sind. Ein Hinweis auf die Lösung der spezifischen Probleme bei der Analyse von Ionen, um die sich die Fachwelt seit vielen Jahren bemüht, ist diesen Druckschriften jedoch nicht zu entnehmen.

Die erfindungsgemäße Testschicht, die auch als Filmschicht bezeichnet werden kann, enthält einen flüssigkeitsfesten Film aus einem hydrophoben Polymer und darin verteilten Partikeln. Das hydrophobe Polymer ist für die zu untersuchende Flüssigkeit und auch für zu bestimmende Ionen nicht durchlässig. Es sind die Partikel, die ein Eindringen der Probenflüssigkeit in die Testschicht ermöglichen. Die Testschicht insgesamt ist für die zu untersuchende Flüssigkeit nicht durchlässig. Es wird lediglich ein bestimmtes Volumen aufgenommen. Als vorteilhaft einsetzbare hydrophobe Polymere haben sich insbesondere Copolymere des Vinylacetates erwiesen. Besonders vorteilhaft sind Copolymere des

Vinylacetats mit Vinyllaurat oder Maleinsäuredibutylester.

Als Partikel können feste, in den zu untersuchenden Flüssigkeiten unlösliche, inerte, anorganische oder organische Partikel eingesetzt werden, die eine Ölzahl von 80 - 200, vorzugsweise 100 - 170 aufweisen. Vor allem haben sich die verschiedenen Arten von Diatomeenerde, wie ungeglühte oder Naturkieselgur, calcinierte oder gebrannte Kieselgur, flußgeglühte oder aktivierte Kieselgur als vorteilhaft für die erfindungsgemäße Filmschicht erwiesen.

Die Ölzahl ist eine aus dem Gebiet der Lacke und Beschichtungen bekannte Kenngröße für Teilchen, die beispielsweise als Füllstoffe eingesetzt werden. Sie ist ein Maß für die Wechselwirkung zwischen den Teilchen und dem Medium in dem sie dispergiert werden. Die Ölzahl ist einfach zu ermitteln. Die Bestimmung erfolgt nach DIN 53 199. Nach dieser Norm gibt die Ölzahl die Menge Leinöl in g an, die gebraucht wird, um 100 g der interessierenden Partikel zu einer zusammenhängenden kittartigen Masse zu verarbeiten. Hierzu werden die Teilchen (Einwaage a in g) auf eine Glasplatte gebracht. Aus einer Bürette werden etwa 2/3 der benötigten Menge Leinöl zugegeben. Öl und Teilchen werden durch intensives Reiben mit dem Spatel unter Druck verarbeitet, bis die Masse homogen ist. Dann wird Öl weiter tropfenweise zugegeben und jeweils in gleicher Weise verarbeitet, bis eine zusammenhaftende, kittartige Masse, die auf der Glasplatte gerade noch nicht schmiert, entstanden ist. Die hierfür verbrauchte Menge Öl b in ml wird auf 0,01 ml gemessen. Die Ölzahl berechnet sich dann nach folgender Formel:

$$\text{Ölzahl} = \frac{0{,}93 \text{ x b x } 100}{a}$$

Hierin bedeutet 0,93 die Dichte des Leinöls in g/ml.

Die eingesetzten Teilchen haben in der Regel eine ungleichmäßige Gestalt. Ihre Korngröße liegt üblicherweise zwischen 0,1 und 200 µm, vorzugsweise zwischen 0,2 und 30 µm. Insbesondere zeichnen sich die erfindungsgemäßen Partikel dadurch aus, daß sie Hohlräume aufweisen, in die Gase und benetzende Flüssigkeiten eindringen können. Diese Eigenschaft drückt sich insbesondere in dem niedrigen Schüttgewicht von 50 bis 250, vorzugsweise 80 bis 180 g/l aus.

Für die erfindungsgemäße Testschicht ist ein Gewichtsverhältnis von hydrophobem Polymer zu Partikel von etwa 5:1 bis etwa 1:10 brauchbar. Vorteilhafterweise beträgt das Gewichtsverhältnis etwa 1:1 bis etwa 1:3. In jedem Fall ist das optimale Gewichtsverhältnis von hydrophobem Polymer zu Partikel von der Natur des eingesetzten Polymers und der Partikel abhängig. Ist das hydrophobe Polymer ein Copolymer des Vinylacetats mit Vinyllaurat und/oder Maleinsäuredibutylester und die Teilchen Diatomeenerde liegt das optimale Gewichtsverhältnis zwischen 1:1,5 und 1:2,5.

Weitere notwendige Bestandteile der erfindungsgemäßen Testschicht sind eine schwer flüchtige mit Wasser nicht mischbare Flüssigkeit und ein Ionophor. Diese Komponenten liegen in homogener Verteilung in dem hydrophoben Polymer vor.

Unter einer schwer flüchtigen, mit Wasser nicht mischbaren Flüssigkeit wird ein Weichmacher für Kunststoffe verstanden. Er dient zusammen mit dem Polymer als organische Phase für das Ionenbestimmungsverfahren. Es kommen alle möglichen handelsüblichen Typen für Kunststoffweichmacher, vorzugsweise Sebacinsäure-, Acrylsäure-, Phthalsäure- und Phosphorsäure-Ester sowie Silicone in Frage. Insbesondere aus verarbeitungstechnischen Gründen wird dem sehr schwer flüchtigen Uvinul®N539 (2,2-Diphenyl-1-cyano-acrylsäureethylhexylester) der Vorzug gegeben.

Das Gewichtsverhältnis von hydrophobem Polymer zu schwer flüchtiger, hydrophober organischer Flüssigkeit in der Testschicht kann zwischen etwa 5:1 bis etwa 1:5, insbesondere etwa 2:1 bis etwa 1:2 betragen.

Als Ionophore sind alle die Ionen komplexierenden Substanzen verwendbar, die für die zu bestimmenden Ionen spezifisch und hinreichend löslich in nicht wässriger Phase sind. Hierbei ist insbesondere an Kronenäther, Kryptanden, Podanden und entsprechende Peptide cyclischer oder acylischer Natur zu denken. Für die Bestimmung von Kalium hat sich 2,3-Naphthol-15-Krone-5 als vorteilhaft erwiesen. Ganz besonders bevorzugt ist das Naturstoffionophor Valinomycin. Für die Bestimmung von Natrium kommen beispielsweise N,N'-Dibenzyl-N,N'-diphenyl-1,2-phenylendioxydiacetamid, für Lithium N,N'-Diheptyl-5,5-dimethyl-N,N'-di(3-oxapentyl)-3,7-dioxanonandiamid und für Calcium Diethyl-N,N'-[(4R,5R)-4,5-dimethyl-1,8-dioxo-3,6-dioxa-octamethylen]-bis-(12-methylaminododecanoat) in Frage.

Der erfindungsgemäße Testträger enthält als wesentlichen Bestandteil eine Substanz, die in Anwesenheit des zu bestimmenden Ions ihre Farbe ändert. Entsprechende mögliche Substanzen wurden einleitend bereits angesprochen. Solche Stoffe können grundsätzlich in der erfindungsgemäßen Testschicht oder in einer zweiten Testschicht vorliegen. Im Falle der zweiten Alternative müssen die beiden Schichten jedoch miteinander in einen Flüssigkeitsaustausch ermöglichenden direkten oder indirekten Kontakt gebracht werden können. Während eine Gegenionfarbsubstanz vorzugsweise nicht in der erfindungsgemäßen Testschicht, sondern in einer anderen Schicht untergebracht ist, kann ein Chromoionophor auch in der erfindungsgemäßen Testschicht selbst vorliegen. Ein pH-Indikator wird in der erfindungsgemäßen Testschicht untergebracht sein. Vorzugsweise wird er dort ebenso wie der Ionophor in der hydrophoben organischen Phase homogen verteilt sein. Die Verwendung von pH-Indikatoren nach dem Prinzip der heterogenen pH-Reaktion ist erfindungsgemäß besonders bevorzugt. Dieses Prinzip kann wie folgt skizziert werden:

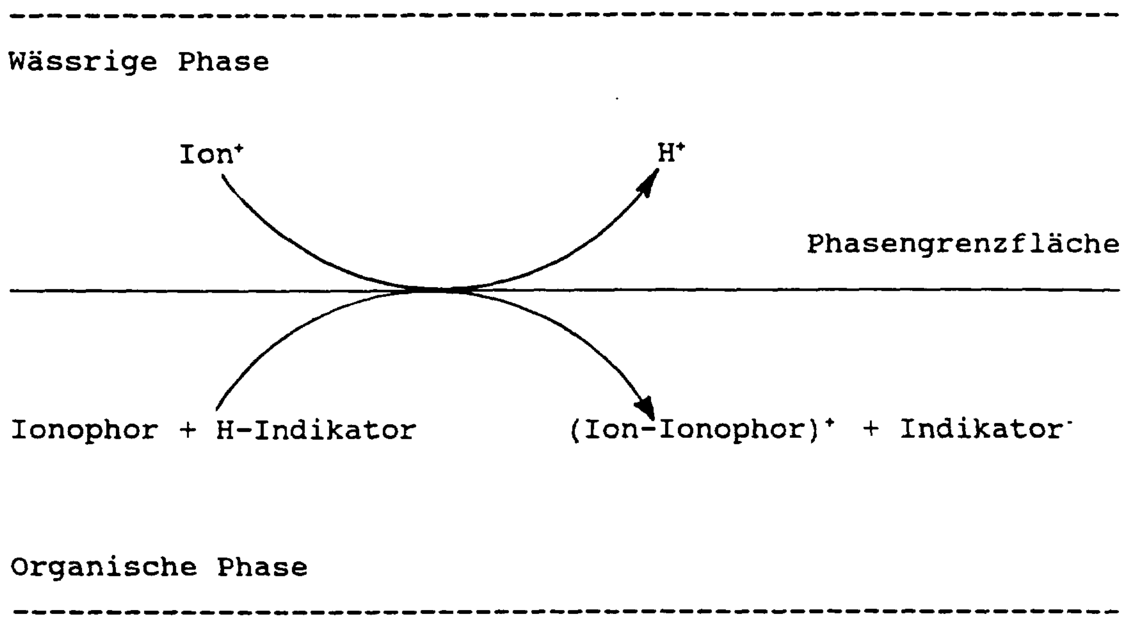


Ionophore sind wie vorstehend bereits beschrieben Komplexbildner für Ionen, die für das genannte Prinzip in organischen Substanzen, die nicht wasserlöslich sind, löslich sein müssen. Als H-Indikator kommen übliche organolösliche pH-Indikatoren infrage. Ionophor und pH-Indikator können auch kovalent verbunden als sogenannter Chromoionophor vorliegen. Das Prinzip funktioniert wie folgt: Ein Ion wird aus der zu untersuchenden wässrigen Phase mit Hilfe des Ionophors in die organische, nicht mit Wasser mischbare, Phase hineingezogen. Zum Ladungsausgleich muß ein Proton in die wässrige Phase abgegeben werden. Dieses Proton wird dem pH-Indikator entnommen, der daraufhin ein farbiges Anion bildet.

Dieses Prinzip ist erstmalig bei E.S.Hyman, Biophysical Society Abstracts, 1971, 72a erwähnt und zwar mit Valinomycin als Ionophor und Tetrabromphenolphthaleinäthylester als pH-Indikator. Beschreibungen mit Chromoionophoren finden sich beispielsweise in K.Ueno und M.Tagaki, Studies in Physical and Theoretical Chemistry 27, 279-293, (1982) sowie H.Nakamura et al., Bunseki Kagaku 31, E131-E134 (1982).

Erfindungsgemäß können als pH-Indikatoren solche eingesetzt werden, die in nicht wässrigen Medien löslich und so hydrophob sind, daß sie unter Analysebedingungen nicht aus der organischen Phase in eine wässrige Phase herausextrahiert werden. Der pK-Wert sollte so sein, daß gemäß dem skizzierten Prinzip zur Bestimmung von Ionen unter Analysebedingungen in Anwesenheit des zu bestimmenden Ions die Indikatorsubstanz Protonen abgeben kann und dadurch eine Änderung seines farblichen Zustandes erfährt. Brauchbar sind z. B. Tetrabromphenolphthaleinester, wie in E.S.Heyman, Biophysical Society Abstracts, 1971, 72a beschrieben oder Alkylindonaphthole, wie sie in EP-A-0 128 317 und EP-A-0 128 318 offenbart sind.

Es können auch Chromoionophore als eine kovalent gebunden vorliegende Kombination eines Ionophors und eines pH-Indikators eingesetzt werden. Für die Bestimmung von Kalium hat sich beispielsweise 4'-(2",4"-Dinitro-6"-trifluorphenylmethyl)-aminobenzo-18-krone-6 als vorteilhaft erwiesen. Für die Bestimmung von Natrium kann die entsprechende 15-Krone-5-Verbindung eingesetzt werden.

Bevorzugt wird als pH-Indikator ein Naphtholderivat der allgemeinen Formel I

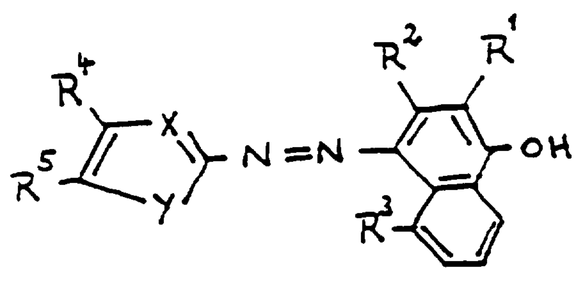

(I)

in der

$R^1$, $R^2$, $R^3$ gleich oder verschieden sind und Wasserstoff, eine Alkyl- oder Alkoxygruppe darstellen, wobei mindestens einer der Reste ein ($C_8$ -$C_{30}$)-Alkyl- oder Alkoxyrest ist,

| | |
|---|---|
| $R^4$ | Wasserstoff oder eine Alkylgruppe, |
| $R^5$ | eine Nitrogruppe, eine durch Halogen substituierte Alkylgruppe, eine Cyanogruppe, eine Sulfonamidgruppe oder eine Alkylsulfonylgruppe, |
| X | Stickstoff oder den Rest $CR^6$ und |
| Y | Schwefel oder den Rest $CR^7 = CR^8$ |

wobei $R^6$, $R^7$, $R^8$ gleich oder verschieden sind und
Wasserstoff, Halogen, eine Nitrogruppe, eine Cyanogruppe, eine Alkyl- oder durch Halogen substituierte Alkylgruppe oder eine Alkylsulfonylgruppe
bedeuten, eingesetzt. Solche Substanzen sind in DE-A-4015591.9 beschrieben.

Unter einer Alkylgruppe in der Definition der Reste $R^1$, $R^2$, $R^3$, $R^4$, $R^6$, $R^7$ und $R^8$ wird ein Alkylrest mit 1 bis 30 Kohlenstoffatome aufweisender Alkylrest verstanden. Insbesondere für die Reste $R^4$, $R^6$, $R^7$ und $R^8$ sind Alkylreste mit 1 bis 4 Kohlenstoffatomen, insbesondere 1 bis 2 Kohlenstoffatome, bevorzugt. Was die Reste $R^1$, $R^2$ und $R^3$ anbelangt, so ist vorzugsweise nur einer der Reste ein Alkylrest mit 8 bis 30, vorzugsweise 10 bis 20 Kohlenstoffatomen. Sollen die übrigen Reste dieser Gruppe ebenfalls eine Alkylgruppe darstellen, so sind sie vorzugsweise ein Alkylrest mit 1 bis 4, vorzugsweise 1 bis 2 Kohlenstoffatomen. Alkylreste mit mehr als 2 Kohlenstoffatomen können geradkettig oder verzweigt sein. Außerdem kann der Alkylrest auch teilweise ungesättigt sein.

Unter einer durch Halogen substituierten Alkylgruppe in der Definition von $R^5$, $R^6$, $R^7$ und $R^8$ wird ein durch Fluor, Chlor, Brom oder Jod substituierter Alkylrest mit 1 bis 4 Kohlenstoffatomen verstanden. Bevorzugt sind durch Fluor substituierte Alkylreste mit 1 bis 2 Kohlenstoffatomen. Besonders bevorzugt ist der Trifluormethylrest.

Eine Alkoxygruppe in der Definition der Reste $R^1$, $R^2$, $R^3$ ist ein Alkoxyrest mit 8 bis 30, vorzugsweise 10 bis 20 Kohlenstoffatomen. Der Alkoxyrest kann geradkettig oder verzweigt, gesättigt oder teilweise ungesättigt sein.

Halogen in der Definition der Reste $R^6$, $R^7$, $R^8$ kann Fluor, Chlor, Brom oder Jod bedeuten, bevorzugt sind Chlor und Brom.

Eine Alkylsulfonylgruppe in der Definition der Reste $R^5$, $R^6$, $R^7$, $R^8$ bedeutet die Gruppierung Alkyl-$SO_2$-. In diesem Zusammenhang stellt die Alkylgruppe einen Alkylrest aus 1 bis 4, vorzugsweise 1 bis 2 Kohlenstoffatomen dar. Besonders bevorzugt ist die Methylsulfonylgruppe.

Unter einer Sulfonamidgruppe in der Definition des Restes $R^5$ wird ein unsubstituiertes Amid ($-SO_2NH_2$) oder ein Amid eines primären oder sekundären Amins ($-SO_2NHR$ oder $-SO_2NR_2$) verstanden. Substituenten des Amids (R) können Alkyl, Aryl oder Aralkylreste sein. Im Falle des Amids eines sekundären Amins können die Substituenten (R) gleich oder verschieden sein. Unter einem Alkylrest wird in diesem Zusammenhang ein Rest mit 1 bis 4 Kohlenstoffatomen verstanden. Ein Arylrest bedeutet einen aromatischen Rest mit 6 bis 10 Kohlenstoffatomen. Bevorzugte Arylreste sind der Phenyl- oder Naphthylrest. Aralkylreste sind solche Reste, in denen der Arylteil ein aromatischer Rest mit 6 bis 10 Kohlenstoffatomen und der Alkylteil ein Rest mit 1 bis 4 Kohlenstoffatomen ist. Bevorzugter Aralkylrest ist der Benzylrest. Für die vorliegende Erfindung ist die unsubstituierte Sulfonamidgruppe ($-SO_2NH_2$) besonders bevorzugt.

Besonders bevorzugte Naphtholderivate der allgemeinen Formel I sind solche, bei denen einer der Reste $R^1$, $R^2$ und $R^3$ einen Alkyl oder Alkoxyrest mit 8 bis 30, vorzugsweise 10 bis 20 Kohlenstoffatomen darstellt und die übrigen Reste aus der vorgenannten Gruppe Wasserstoff oder einen Alkylrest mit 1 bis 4, vorzugsweise 1 bis 2 Kohlenstoffatomen bedeuten.

Ganz besonders bevorzugte Naphtholderivate sind solche, in denen $R^I$ eine Alkoxygruppe mit 10 bis 20 Kohlenstoffatomen, $R^2$ und $R^3$ Wasserstoff darstellen und die übrigen Reste die für Formel I angegebene Bedeutung haben.

Zur Herstellung von Naphtholderivaten der allgemeinen Formel II sind mehrere Verfahrensvarianten möglich. Zum einen können Naphthochinone der allgemeinen Formel II

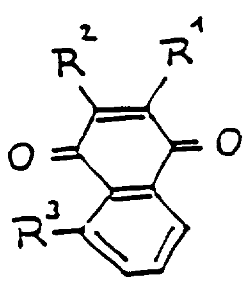

(II)

in der
$R^1$, $R^2$ und $R^3$ die für die allgemeine Formel I angegebene Bedeutung haben, mit einem Hydrazin der allgemeinen Formel III

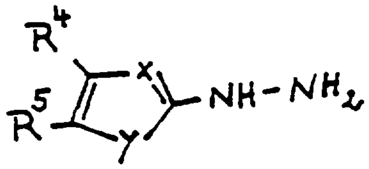

(III)

in der $R^4$, $R^5$, X und Y die für die allgemeine Formel I angegebene Bedeutung haben umgesetzt werden. Diese Umsetzung kann unter den für die Bildung eines Hydrazons üblichen Bedingungen erfolgen. Vorzugsweise erfolgt die Reaktion unter sauren Bedingungen. Das Hydrazon selbst ist nicht beständigt, sondern lagert sich zu dem gewünschten Naphthol der allgemeinen Formel I um.

Ein anderer Weg zur Herstellung der erfindungsgemäßen Naphtholderivate der allgemeinen Formel I geht aus von Aminen der allgemeinen Formel IV

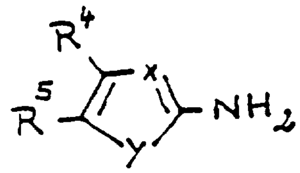

(IV)

in der $R^4$, $R^5$, X und Y die für die Formel I angegebene Bedeutung haben. Diese Amine werden diazotiert und die resultierenden Diazoniumsalze mit einem Naphthol der allgemeinen Formel V

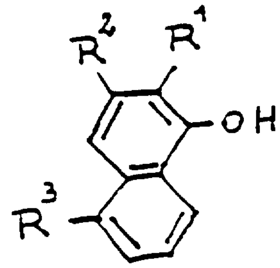

(V)

in der $R^1$, $R^2$ und $R^3$ die für die allgemeine Formel I angegebene Bedeutung haben, in einer Azokupplungsreaktion umgesetzt.

Die Diazotierung der Amine der allgemeinen Formel IV kann in der üblichen Weise erfolgen. Als vorteilhaft erwiesen hat es sich, konzentrierte Mineralsäure, beispielsweise konzentrierte Schwefelsäure mit einem Nitrit, vorzugsweise Natriumnitrit vorzulegen und unter Kühlung auf Raumtemperatur das Amin der allgemeinen Formel IV zuzugeben. Als ganz besonders vorteilhaft hat sich eine Diazotierungsmischung erwiesen, die Natriumnitrit und neben konzentrierter Schwefelsäure auch Eisessig enthält. Das bevorzugte Volumenverhältnis von Schwefelsäure und Eisessig liegt zwischen 1:1 und 2:1. Üblicherweise ist das Verhältnis von Nitrit und zu diazotierendem Amin der allgemeinen Formel IV äquimolar.

Nach beendeter Diazotierungsreaktion wird die Reaktionsmischung wässrig aufgearbeitet. Vorteilhafterweise gießt man hierzu die Reaktionsmischung auf Eiswasser. Das Diazoniumsalz selbst wird nicht isoliert, sondern in der wässrigen Aufarbeitungslösung mit dem Naphthol der allgemeinen Formel V zur Azokupplung gebracht. Diese erfolgt vorzugsweise unter schwach sauren Bedingungen. Naphthole der allgemeinen Formel V sind in wässrigen Lösungen nur sehr schwer löslich. Sie werden deshalb in organischen Lösungsmitteln zum Einsatz gebracht. Als organisches Lösungsmittel ist beispielsweise Chloroform gut geeignet. So kann eine nach wässriger Aufarbeitung vorliegende Diazoniumsalzlösung zu einer Lösung eines Naphthols der allgemeinen Formel V in Chloroform und Eisessig und Zugabe eines Azetats zur Abpufferung des pH-Wertes des Reaktionsmediums hinzugefügt werden. Meist fallen die gebildeten Naphtholderivate der allgemeinen Formel I aus der Reaktionsmischung aus. Das Produkt kann dann umkristallisiert oder chromatographisch aufgereinigt werden.

Insbesondere bei Einsatz von pH-Indikatoren ist es aufgrund deren naturgemäßer Empfindlichkeit gegen pH-Änderungen vorteilhaft, in die erfindungsgemäße Testschicht auch einen Puffer einzubringen. Der pH-Wert eines Puffers regelt bei Bestimmungsverfahren für Ionen, die auf dem auf Seite 9 dargestellten Prinzip der heterogenen pH-Reaktion beruhen, den Übertritt des Protons aus der nichtwässrigen in die wässrige Phase. Er richtet sich daher nach dem pK-Wert des verwendeten pH-Indikators. Vorzugsweise wählt man bei diagnostischen Mitteln für die Bestimmung von Ionen in Körperflüssigkeiten die Puffersubstanz so, daß ein pH-Wert zwischen 5 - 10, vorzugsweise 7 bis 8, eingestellt werden kann. Hierfür kommen prinzipiell alle Puffer in Frage, sofern sie wasserlöslich sind und keine Ionen enthalten, die die Bestimmungsreaktion stören. Bewährt haben sich Puffer aus der Reihe der sogenannten Good-Puffer, wie z. B. N,N-Bis-(hydroxyethyl)-aminoethansulfonsäure (BES), 3-[N-Trishydroxymethyl]-methylamino-hydroxypropansulfonsäure (TAPSO), oder N-Hydroxyethylpiperazin-N-propansulfonsäure (HEPPS).

Werden Ionophore verwendet, die nicht genügend selektiv für das zu bestimmende Ion sind, so können wasserlösliche Komplexbildner zugesetzt werden, die störende Ionen maskieren. So wird z. B. eine etwaige Störung eines Natriumtestes durch Calcium mit Ethylendiamintetraacetat (EDTA) verhindert.

Weiter können zur Verbesserung der Testschichtherstellung oder der Testschichtbenetzung durch die zu untersuchende Probe Netzmittel eingesetzt werden. Hierzu sind nur solche verwendbar, die die Nachweisreaktion nicht stören. Dies sind insbesondere nichtionische und zwitterionische Verbindungen. Von den nichtionischen Netzmitteln haben sich beispielsweise Polyethylenglykolether oder -ester, vorzugsweise Triton® X100 als vorteilhaft erwiesen. Als zwitterionisches Netzmittel kann vorteilhafterweise n-Decyl-N,N-dimethyl-3-ammonio-1-propansulfonat (Zwittergent®3-10) eingesetzt werden.

Zur Verbesserung der Konsistenz der erfindungsgemäßen Testschicht können zusätzlich auch Verdicker eingesetzt werden. Als besonders vorteilhaft hat sich hier Ethylcellulose erwiesen. Für die nach der Benetzung der erfindungsgemäßen Filmschicht mit einer zu analysierenden wässrigen Flüssigkeit vorliegende wässrige Phase kann der erfindungsgemäßen Testschicht auch noch zusätzlich hydrophiles Verdickungsmittel, wie beispielsweise Hydroxyethyl- oder Hydroxypropylcellulose zugegeben werden.

Zur Herstellung einer erfindungsgemäßen Testschicht werden alle Komponenten, die bei einem späteren Einsatz der Testschicht zur Bestimmung eines Ions in einer wässrigen Flüssigkeit, insbesondere einer Körperflüssigkeit, wie Blut, Plasma, Serum oder Urin, nicht in die wässrige Phase aufgenommen werden, sondern in der organischen Phase, d. h. der Filmschicht bleiben (hydrophobes Polymer; schwer flüchtige, mit Wasser nicht mischbare Flüssigkeit; Ionophor; gegebenenfalls Chromogen, wie pH-Indikator und gegebenenfalls Verdicker zur Verbesserung der Konsistenz der Filmschicht) in einem leicht- bis mittelflüchtigen organischen Lösungsmittel gelöst. In diese Lösung werden die Partikel eingerührt und darin homogen verteilt. Die Masse wird danach auf einer Unterlage mit einem Rakel ausgestrichen und getrocknet. Es können naturgemäß auch andere geeignete Auftragsverfahren, wie Walzenauftrag, Filmgießen oder ähnliche angewendet werden. Die trockene Filmschicht hat eine Dicke von 20 bis 500, vorzugsweise von 20 bis 150 µm.

Zur Einbringung von Bestandteilen, die bei Aufgabe der wässrigen Probenflüssigkeit auf die erfindungsgemäße Filmschicht in die wässrige Phase aufgenommen werden (Puffer; gegebenenfalls Komplexbildner; gegebenenfalls Netzmittel; gegebenenfalls Verdicker zur Veränderung der Konsistenz der wässrigen Phase) gibt es verschiedene Wege. Eine Möglichkeit ist es, die Partikel mit den genannten Komponenten zu belegen, indem man die Teilchen zusammen mit einer wässrigen Lösung der Komponenten eindampft, sprühtrocknet oder gefriertrocknet. Die so belegten Partikel werden dann wie oben beschrieben in die organische Lösung eingerührt. Eine andere Möglichkeit besteht darin, die Filmschicht erst mit unbehandelten Partikeln herzustellen, dann mit einer wässrigen Lösung der vorstehend genannten Bestandteile nachzubeschichten und abschließend zu trocknen.

Die Filmschicht wird vorzugsweise mit der Unterlage als Träger zusammen verwendet werden. Insbesondere kann die erfindungsgemäße Filmschicht zu bekannten Einsatzformen weiterverarbeitet werden, wie sie schon vielfach für Schnelldiagnostica beschrieben sind. Es sind dies zum Beispiel einfache Testträger, bei denen der Nachweisfilm an das eine Ende eines schmalen, steifen Streifens, beispielsweise aus Kunststoff, aufgeklebt ist.

Für einen Testträger, der neben der erfindungsgemäßen Testschicht noch eine oder mehrere andere Schichten enthält, kann es auch vorteilhaft sein, Komponenten, die während der Bestimmungsreaktion in der wässrigen Phase aufgenommen werden können, nicht in der erfindungsgemäßen Filmschicht vorzulegen, sondern diese vollkommen von ihr zu separieren. Dies kann beispielsweise dazu führen, daß man diese Komponenten auf eine andere Trägerschicht aufbringt, beispielsweise eine Papierschicht oder ein Netz.

Die Auswertung eines Testes mittels eines erfindungsgemäßen Testträgers kann sowohl visuell als auch auf bekannte Weise reflexionsphotometrisch erfolgen.

Für die Richtigkeit insbesondere der reflexionsphotometrischen Auswertung hat es sich als besonders vorteilhaft erwiesen, wenn die Filmschicht eine in nicht wässrigem Medium gut lösliche organische Säure enthält. Ein solches Verfahren ist in DE-A-4015592.7 beschrieben. Als ganz besonders vorteilhaft haben sich höher nitrierte Alkylphenole bzw. höher nitrierte Alkoxynaphthole oder Carbonyldicyanidphenylhydrazone erwiesen. Solche Substanzen sind in der Lage, den Bereich der größten Meßempfindlichkeit mit dem relevanten Konzentrationsbereich des zu bestimmenden Ions in Übereinstimmung zu bringen. Hervorragend bewährt haben sich in diesem Zusammenhang Bis-(2-hydroxy-3,5,6-trichlorphenyl)-methan (Hexachlorophen), 3-Pentadecyl-2,4,6-trinitrophenol und 2,4,6,8-Tetranitro-5-octadecyloxy-naphthol-1 sowie [(2,4-Dinitrophenyl) hydrazono]propandinitril (Mesoxalsäuredinitril-2,4-dinitrophenylhydrazon).

Die folgende Tabelle 1 gibt die vorteilhaften und bevorzugten Gewichtsprozente der Bestandteile einer erfindungsgemäßen Testschicht an:

Tabelle 1:

| Bestandteil der Filmschicht | Gehalt der Filmschicht in Gew.-% | |
|---|---|---|
| | vorteilhaft | bevorzugt |
| Polymer | 5 - 60 | 20 - 40 |
| schwerflüchtige, mit Wasser nicht mischbare Flüssigkeit | 5 - 70 | 20 - 40 |
| Partikel | 15 - 80 | 30 - 50 |
| Ionophor | 0,05 - 5,0 | 0,2 - 1,0 |

Ist Puffersubstanz in oder auf die erfindungsgemäße Filmschicht gebracht, so enthält diese 5 - 30, vorzugsweise 10 - 20 Gew.-% Puffer. Wenn Chromogen, wie beispielsweise ein pH-Indikator in der erfindungsgemäßen Testschicht enthalten ist, dann liegt er dort zu etwa 0,05 - 5,0, vorzugsweise 0,2 - 0,7 Gew.-% vor. Die gegebenenfalls einsetzbaren Substanzen, wie Komplexbildner, Netzmittel, Verdicker oder Säure zur Empfindlichkeitssteigerung, liegen - falls sie in die erfindungsgemäße Filmschicht ein oder aufgebracht worden sind - in Mengen von 0,005 bis 5, vorzugsweise 0,02 bis 2 Gew.-% der erfindungsgemäßen Filmschicht vor.

In den Fig. 1 und 2 sind besonders vorteilhafte Ausgestaltungsformen eines erfindungsgemäßen Testträgers zur Bestimmung von Ionen in Flüssigkeiten dargestellt, die eine erfindungsgemäße Filmschicht beinhalten. Es zeigen:

Fig. 1 einen besonders bevorzugten Testträger mit einer erfindungsgemäßen Filmschicht.

Fig. 2 einen weiteren besonders bevorzugten Testträger mit einer erfindungsgemäßen Filmschicht.

In den Figuren 1 und 2 sind zwei Testträger räumlich dargestellt, die für die Bestimmung von Ionen in Blut geeignet sind. Sie erlauben, aus Vollblut Serum bzw. Plasma abzutrennen und in der so gewonnenen Flüssigkeit die Bestimmung der interessierenden Ionen durchzuführen. Die Testträger unterscheiden sich im wesentlichen nur durch die Anordnung der Puffersubstanz innerhalb des Testträgers. Im einzelnen sind die Vorrichtungen wie folgt zusammengesetzt:

Fig. 1: Auf einer inerten Trägerfolie (5), beispielsweise einer Kunststoffolie, ist eine Transportschicht (2) befestigt, die zum Transport der Probenflüssigkeit aus der Probenaufgabezone (7) in den Nachweisbereich (8) dient. Als Transportschicht (2) ist prinzipiell jedes Material geeignet, das die zu untersuchende Flüssigkeit aus der Probenaufgabezone (7) in den Nachweisbereich (8) zu transportieren in der Lage ist und sie dabei nicht so verändert, daß die Analyse beeinträchtigt wird. Besonders vorteilhaft ist es, als Transportschicht (2) ein Glasfaservlies einzusetzen. Die Transportschicht (2) teilweise überdeckend ist eine Schicht (3) zur Abtrennung von korpuskulären Bestandteilen aus der Probenflüssigkeit befestigt. Grundsätzlich kann hierfür jedes Material verwendet werden, das erlaubt, korpuskuläre Bestandteile aus der Probenflüssigkeit, insbesondere Blutzellen, vor allem Erythrozyten aus Blut, abzutrennen und diese nicht in wesentlichen Mengen in den Nachweisbereich (8) gelangen zu lassen, so daß sie dort keine Störung der Nachweisreaktion verursachen. Im übrigen darf die Abtrennschicht (3) nicht zu einer Veränderung der Probenflüssigkeit dahingehend führen, daß darin die Konzentration der zu bestimmenden Ionen verändert und so das Ergebnis verfälscht wird. Als besonders geeignet für die Abtrennschicht (3) haben sich Glasfaservliese erwiesen, wie sie z. B. in EP-B-0 045 476 beschrieben sind. Über der Abtrennschicht (3) ist eine Schutzschicht (4) angebracht, die verhindern soll, daß bei der Probenaufgabe, beispielsweise mittels einer Pipette, die Abtrennschicht (3) beschädigt wird. Bewährt hat sich hierfür ein Netz aus inertem Material, beispielsweise aus Kunststoff. Schutzschicht (4) und Abtrennschicht (3) sind auf der inerten Trägerfolie (5) befestigt. Dies kann beispielsweise mittels eines Schmelzklebestreifens (6) erfolgen.

Seitlich von der Transportschicht (2) ist eine aus durchsichtigem Kunststoff bestehende Trägerfolie mit der erfindungsgemäßen Testschicht (1) befestigt. Vorteilhafterweise geschieht dies über eine Klebestelle (9), beispielsweise einen Schmelzkleberstreifen. Die Filmschicht (1) ist so angeordnet, daß sie beim Niederdrücken der durchsichtigen Trägerfolie in Richtung der inerten Trägerfolie (5) mit der Transportschicht (2) in einen einen Flüssigkeitsübergang ermöglichenden Kontakt gebracht werden kann.

Fig. 2 unterscheidet sich von Fig. 1 darin, daß ebenfalls über die Klebestelle (9), beispielsweise einen Schmelzkleberstreifen, zwischen Filmschicht (10) und Transportschicht (2) eine Schicht (11) angebracht ist, die solche Substanzen enthält, die während der Bestimmungsreaktion in die wässrige Phase aufgenommen werden. Solche Substanzen sind insbesondere Puffersubstanzen. Aber auch Komplexbildner, Netzmittel oder Verdicker zur Veränderung der Konsistenz der wässrigen Phase können statt in der erfindungsgemäßen Filmschicht (1) des Testträgers gemäß Fig. 1 oder (10) des Testträgers gemäß Fig. 2 in der zusätzlichen Schicht (11) des Testträgers gemäß Fig. 2 untergebracht sein. Als Material für die zusätzliche Schicht (11) kommen saugfähige Materialien in Frage, die einen Flüssigkeitsübergang auf eine weitere Schicht ermöglichen, wenn diese in Kontakt mit ihr gebracht wird. Besonders vorteilhaft ist Papier einsetzbar, aber auch Netze oder Gewebe aus inertem Material, wie Kunststoff sind möglich.

Zur Durchführung der Bestimmung eines Ions in Blut mittels eines der in den Figuren dargestellten Testträgers wird die Probe auf die Schutzschicht (4) gegeben. Das Blut dringt in die Abtrennschicht (3) ein und Erythrozyten werden von Plasma bzw. Serum getrennt. Durch Kapillarkräfte wird die so gewonnene Flüssigkeit in die Nachweiszone (8) gesaugt.

Durch Druck auf die Trägerfolie mit der erfindungsgemäßen Filmschicht (1) bzw. (10) wird die wässrige Phase in der Transportschicht (2) mit der erfindungsgemäßen Filmschicht in Kontakt gebracht, Flüssigkeit dringt in die Filmschicht ein und die Bestimmungsreaktion wird ausgelöst. Die Reaktion wird anhand der Verfärbung in der Filmschicht durch die Trägerfolie der Filmschicht (1) bzw. (10) hindurch visuell beobachtet oder reflexionsphotometrisch gemessen.

In den folgenden Beispielen wird die Erfindung weiter erläutert.

Beispiel 1

Allgemeine Herstellvorschrift für Testträger mit erfindungsgemäßer Filmschicht

Zur Herstellung eines Testträgers gemäß Fig. 1 wird durchsichtige Polyesterfolie (200 µm dick) mit den in den nachfolgenden Beispielen genannten Mischungen beschichtet und getrocknet. Die beschichtete Folie wird in 15 mm breite Streifen geschnitten und mittels Schmelzkleber als Schicht (1) längs auf 150 mm breite weiße Polyesterfolie (5) geklebt. Ebenso werden Streifen von Glasfaservlies mit einem Flächengewicht von 30 $g/m^2$ als Transportschicht (2), Glasfaservlies mit einem Flächengewicht von 60 $g/m^2$ als Abtrennschicht (3) und Polyamidgewebe als Schutzschicht (4) längs auf diese weiße Polyesterfolie geklebt, so daß nach Querschneiden 6 mm breite Teststreifen gemäß Fig. 1 entstehen.

Testträger gemäß Fig. 2 werden analog hergestellt. Die Schicht (11) besteht aus Filterpapier, das mit Puffersubstanz imprägniert ist.

Die erfindungsgemäße Filmschicht-bzw. Testträger werden so eingesetzt, daß auf das Polyamidgewebe (4) 30 µl der zu untersuchenden Probe aufgebracht und der Testträger danach in das handelsübliche Reflexionsphotometer Reflotron® (Boehringer Mannheim GmbH, Mannheim, Bundesrepublik Deutschland) eingeführt wird. Die Flüssigkeit dringt in das Glasfaservlies (3) ein, wo im Falle von Vollblut die Erythrozyten abgetrennt werden und gelangt in die als Transportschicht dienende Glasfaserzone (2). Im Reflexionsphotometer wird der erfindungsgemäße Film unter der Klappe (1) bzw. (10) durch Druck auf die Klappe mit der Flüssigkeit in der Transportschicht (2) in Berührung gebracht und die entstehende Farbe wird bei 642 nm und 37 °C reflexionsphotometrisch gemessen.

Beispiel 2

Erfindungsgemäße Filmschichten

Es werden Mischungen der nachfolgenden Zusammensetzung hergestellt und mit einer Naßfilmdicke von 300 µm auf durchsichtige Polyesterfolien aufgebracht und getrocknet:

| | |
|---|---|
| 2,2-Diphenyl-1-cyano-acrylsäure-äthylhexylester (Uvinul®N539, BASF, Ludwigshafen, BRD) | 6,42 g |
| 4-(2,6-Dibrom-4-nitro-phenylazo-)-2-octadecyloxynaphthol-1 (Beispiel 5 a - c) | 0,69 g |
| Valinomycin | 0,107 g |
| Diatomeenerde (Celatom® MW 25, Eagle-Picher, Cincinatti, USA) | 10,05 g |
| Methyläthylketon | 18,1 g |
| Vinylacetat-vinyllaurat-Copolymer (Vinnapas® B 500/20 VL, Wacker-Chemie, München, BRD) | 5,24 g |

Auf diese Schichten wird mit einer Naßfilmdicke von 150 µm eine zweite Schicht folgender Zusammensetzung aufgebracht und gleichfalls getrocknet:

| | |
|---|---|
| N,N-Bis-(hydroxyäthyl)-aminoäthansulfonsäure | 81,13 g |
| Hydroxyäthylcellulose (Natrosol® 250G, Hercules Inc., Willmington, Delaware, USA) | 4,81 g |
| Wasser | 236 g |
| Ethanol | 334 g |
| mit LiOH auf pH 7,5 eingestellt | |

Mit der beschichteten Folie werden Testträger gemäß Fig. 1, wie in Beispiel 1 beschrieben, hergestellt und vermessen. Die Messung erfolgt mit Serum mit 5,06 mmol Kalium/l in Abständen von 4 Sekunden nach Berührung der Probe mit der erfindungsgemäßen Filmschicht

Tabelle 2

| Sekunden | % Remission |
|---|---|
| 4 | 24,8 |
| 8 | 21,7 |
| 12 | 20,0 |
| 16 | 19,2 |
| 20 | 18,6 |
| 24 | 18,2 |
| 28 | 17,9 |
| 32 | 17,7 |
| 36 | 17,5 |
| 40 | 17,4 |
| 44 | 17,2 |
| 48 | 17,1 |
| 52 | 17,0 |
| 56 | 16,9 |
| 60 | 16,9 |
| 64 | 16,8 |
| 68 | 16,8 |

Wie man sieht, ist nach 60 Sekunden eine hinreichende Farbkonstanz bei ausreichender Farbtiefe erreicht.

Beispiel 3

Testträger zur Bestimmung von Kalium gemäß Fig. 1

a) Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-vinyllaurat-Copolymer (Vinnapas® B 500/20 VL, Wacker Chemie, München, BRD) | 13,11 g |
| 2,2-Diphenyl-1-cyano-acrylsäure-äthylhexylester (Uvinul®N539, BASF, Ludwigshafen, BRD) | 16,04 g |
| 4-(2,6-Dibrom-4-nitro-phenylazo-)-2-octadecyloxy-naphthol-1 (Beispiel 5 a - c) | 0,173 g |
| 2,4,6,8-Tetranitro-5-octadecyloxy-naphthol-1 (Beispiel 6) | 0,0456 g |
| Valinomycin | 0,2673 g |
| Diatomeenerde (Celatom® MW 25, Eagle-Picher, Cincinatti, USA) | 25,13 g |
| Essigsäurebutylester | 45,17 g |

Auf diese Schicht wird mit einer Naßfilmdicke von 150 µm eine zweite Schicht folgender Zusammensetzung aufgebracht und gleichfalls getrocknet:

| | |
|---|---|
| N,N-Bis-(hydroxyäthyl)-aminoäthansulfonsäure | 81,13 g |
| Hydroxyäthylcellulose (Natrosol® 250G, Hercules Inc., Willmington, Dellaware, USA) | 4,81 g |
| Wasser | 236 g |
| Ethanol | 334 g |
| mit LiOH auf pH 7,5 eingestellt | |

Aus der beschichteten Folie werden Testträger gemäß Fig. 1 wie in Beispielen 1 und 2 beschrieben, hergestellt und vermessen. Die Messung erfolgt 60 sec nach Berührung der Probe mit dem erfindungsgemäßen Reagenzfilm.

Bei der Verwendung von Seren mit verschiedenen Gehalten an Kalium wird folgende Abhängigkeit der Reflektion (%R) vom Kaliumgehalt gefunden:

Tabelle 3

| Kaliumgehalt [mmol Kalium/l] | Reflektion [% R] |
|---|---|
| 0,24 | 60,2 |
| 1,09 | 53,5 |
| 1,87 | 48,0 |
| 3,18 | 40,3 |
| 4,15 | 36,3 |
| 6,08 | 29,9 |
| 8,10 | 25,3 |
| 10,22 | 22,2 |
| 12,10 | 19,8 |

Werden die Messungen mehrfach durchgeführt und die %R-Werte über eine Eichkurve in Konzentrationen umgerechnet, so erhält man Variationskoeffizienten zwischen 1 und 2 %.

b) Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-Maleinsäuredibutylester-Copolymer (Mowilith® 35/73, Hoechst AG, Frankturt, BRD) | 14,7 g |
| 2,2-Diphenyl-1-cyano-acrylsäure-äthylhexylester (Uvinul®539N, BASF, Ludwigshafen, BRD) | 18,4 g |
| 4-(2,6-Dibrom-4-nitro-phenylazo-)-2-octadecyloxynaphthol-1 (Beispiel 5 a - c) | 0,130 g |
| Valinomycin | 0,600 g |
| Diatomeenerde (Celatom® MW 25, Eagle-Picher, Cincinatti, USA) | 28,2 g |
| Essigsäurebutylester | 50,7 g |

Auf diese Schicht wird mit einer Naßfilmdicke von 150 µm eine zweite Schicht folgender Zusammensetzung aufgebracht und gleichfalls getrocknet:

| | |
|---|---|
| Hydroxyäthylcellulose (Natrosol® 250G, Hercules Inc., Willmington, Delaware, USA) in Wasser | 41,5 g |
| N,N-Bis-(hydroxyäthyl)-aminoäthansulfonsäure | 8,5 g |
| Ethanol | 64 ml |
| mit LiOH auf pH 7,8 eingestellt | |

Aus der beschichteten Folie werden Testträger gemäß Fig. 1 wie in Beispielen 1, 2 und 3 a) beschrieben, hergestellt und vermessen. Die Messung erfolgt 60 sec nach Berührung der Probe mit dem Reagenzfilm.

Bei der Verwendung von Seren mit verschiedenen Gehalten an Kalium wird folgende Abhängigkeit der Reflektion (%R) vom Kaliumgehalt gefunden:

Tabelle 4

| Kaliumgehalt [mmol Kalium/l] | Reflektion [% R] |
|---|---|
| 1,00 | 33,4 |

Fortsetzung der Tablelle auf der nächsten Seite

Tabelle 4 (fortgesetzt)

| Kaliumgehalt [mmol Kalium/l] | Reflektion [% R] |
|---|---|
| 1,98 | 28,5 |
| 2,99 | 25,1 |
| 4,12 | 22,7 |
| 6,00 | 19,3 |
| 8,04 | 17,1 |
| 10,12 | 15,4 |
| 11,98 | 14,3 |

Beispiel 4

Testträger zur Bestimmung von Kalium gemäß Fig. 2

Es wird eine Mischung der nachfolgenden Zusammensetzung hergestellt und mit einer Naßfilmdicke von 300 µm auf eine durchsichtige Polyesterfolie aufgebracht und getrocknet:

| | |
|---|---|
| Vinylacetat-vinyllaurat-Copolymer (Vinnapas® B 500/20 VL, Wacker Chemie, München, BRD) | 19,6 g |
| 2,2-Diphenyl-1-cyano-acrylsäure-äthylhexylester (Uvinul®N539, BASF, Ludwigshafen, BRD) | 24,0 g |
| 4-(2-Brom-4-nitro-6-trifluormethylphenylazo-)-2-octadecyloxy-naphthol-1 (Beispiel 5 d) | 0,071 g |
| Mesoxalsäuredinitril-2,4-dinitrophenylhydrazon (Beispiel 8) | 0,052 g |
| Valinomycin | 0,30 g |
| Diatomeenerde (Celatom® MW 25, Eagle-Picher,Cincinatti, USA) | 37,5 g |
| m-Xylol | 67,4 g |

Langfaserpapier 6776 (Schöller und Hösch, Gernsbach, Bundesrepublik Deutschland) wird mit folgender Lösung imprägniert und getrocknet:

| | |
|---|---|
| N,N-Bis-(hydroxyäthyl)-aminoäthansulfonsäure | 8,5 g |
| n-Octylglucosid | 0,1 g |
| Wasser, dest. | 91,5 ml |
| mit LiOH auf pH 7,5 eingestellt | |

Aus der beschichteten Folie (10) und dem imprägnierten Papier (11) werden Testträger gemäß Fig. 2 wie in Beispiel 1 beschrieben, hergestellt und vermessen. Die Messung erfolgt 60 sec nach Berührung der Probe mit dem Reagenzfilm.

Bei der Verwendung von Seren mit verschiedenen Gehalten an Kalium wird folgende Abhängigkeit der Reflektion (%R) vom Kaliumgehalt gefunden:

Tabelle 5

| Kaliumgehalt [mmol Kalium/l] | Reflektion [% R] |
|---|---|
| 0,08 | 63,6 |
| 1,01 | 57,0 |
| 1,98 | 46,3 |
| 3,10 | 37,6 |
| 4,08 | 31,0 |
| 6,08 | 25,0 |
| 8,05 | 22,0 |
| 9,90 | 20,0 |

Beispiel 5

4-[(2,6-Dibrom-4-nitrophenyl)azo]-2-octadecyloxy-1-naphthol

a) 2-Octadecyloxynaphthalin

Man gibt in einen 4 l-Dreihalskolben mit Rührer, Kühler und Thermometer 172,8 g (1,2 mol) 2-Naphthol (98 %) zu einer Lösung von 48 g (1,2 mol) Natriumhydroxid (99 %) in 1 l Ethanol, fügt nach erfolgter Lösung 417 g (1,25 mol) n-Octadecylbromid hinzu und erhitzt die Reaktionsmischung 14 Stunden unter Rückfluß. Nach Zugabe von weiteren 1 l Ethanol wird die heiße Lösung zur Entfernung anorganischen Materials über ein Seitzfilter abgesaugt und das schwach rosa gefärbte Filtrat durch 30minütiges Einstellen in ein Eisbad zur Kristallisation gebracht. Nach Absaugen der fast farblosen Kristalle wäscht man den Filterkuchen portionsweise mit ca. 700 ml Ethanol und erhält nach Trocknen über Diphosphorpentoxid 371,9 g (93,7 % der Theorie) 2-Naphthyl-octadecylether, farblose Kristalle, Fp. 64-68°c.

DC: Kieselgel 60 (Merck), Laufmittel: n-Heptan/Methylethylketon 2:1, $R_f$ = 0,34

b) 2-Octadecyloxy-1-naphthol

In einem 10 l-Dreihalskolben mit Rührer, Claisenaufsatz Thermometer und Kühler mit Chlorcalciumrohr gibt man 594 g (1,5 mol) 2-Octadecyloxynaphthalin und 397 g (0,75 mol) Bleitetraacetat in ein Gemisch von 3 1 Eisessig und 600 ml Acetanhydrid und erwärmt auf 55°C. Während 4 Tagen werden im Zeitabstand von 24 Stunden weitere 400 g (jeweils 100 g) Bleitetraacetat portionsweise unter Rühren zugegeben. Danach wird die entstandene gelbe Lösung auf Raumtemperatur abgekühlt, nach Zugabe von 1,5 l Wasser 30 Minuten nachgerührt, der entstandene Kristallbrei abgesaugt und portionsweise mit 2 l Wasser gewaschen. Das feuchte Rohprodukt wird in 4 l Toluol gelöst und dreimal mit je 1 l Wasser, dreimal mit 1 l gesättigter Natriumhydrogencarbonatlösung und dann abermals mit 3 mal 1 l Wasser durchgeschüttelt. Nach Trocknen der Toluolphase über Natriumsulfat, Absaugen und Einengen erhält man 635 g braunes Rohprodukt, das chromatographisch wie folgt gereinigt wird: Man löst das erhaltene Kristallisat in einer Mischung von 1,3 l Toluol/Isohexan 5:2 und gibt die Lösung auf eine Kieselgel 60 (Merck)-säule, innerer Durchmesser 11,5 cm, Füllhöhe 1,2 m. Als Laufmittel verwendet man Toluol/Isohexan 5:2 und schneidet Fraktionen von ca. 300 ml. Die Fraktionen 9-52 werden vereinigt und bis zur Gewichtskonstanz eingeengt. Man erhält 324,2 g 2-Octadecyloxy-1-naphtholacetat, Fp. 67-68°C. Dieses wird ohne weitere Reinigung in 1,8 l Methanol unter Erwärmen gelöst und auf 20°C abgekühlt. Zu der entstandenen Suspension tropft man innerhalb 15 Minuten ohne Kühlung unter Rühren 93 ml konz. Schwefelsäure zu, wobei die Temperatur auf 35°C ansteigt. Anschließend erhitzt man 2 Stunden unter Rückfluß, kühlt dann mit einem Eisbad und rührt 30 Minuten unter Eiskühlung nach. Die gebildeten Kristalle werden abgesaugt, mit 150 ml eiskaltem Methanol gewaschen und bei 35 °C im Trockenschrank über Diphosphorpentoxid getrocknet. Man erhält 294,4 g (47,5 % der Theorie) 2-Octadecyloxy- 1-naphthol, farblose Kristalle, Fp. 58-59°C.

c) 4-[(2,6- Dibrom-4-nitrophenyl)azo]-2-octadecyloxy-1-naphthol

In einem 2 l Dreihalskolben mit Rührer, Claisenaufsatz und Thermometer werden 22,7 g (0,33 mol) Natriumnitrit während 10-15 Minuten unter Rühren in 300 ml konz. Schwefelsäure eingetragen, wobei man die Temperatur der Reaktionslösung auf 35°C ansteigen läßt. Man kühlt danach auf 20°C und tropft in ca. 15-20 Minuten 230 ml Eisessig so zu, daß die Temperatur unter Eiskühlung bei 20-25°C gehalten wird. Danach gibt man 97,6 ml (0,33 mol) 2,6-Dibrom-4-nitroanilin (Riedel de Haen [99 %GC]) portionsweise während 10 Minuten unter gelegentlichem Kühlen zu, wobei die Temperatur auf19-21°C gehalten wird und rührt noch 3 Stunden nach. Danach gießt man auf 3,5 l Eiswasser und gibt die entstandene Diazoniumsalzlösung rasch zu einer Lösung von 124 g (0,3 mol) 2-Octadecyloxy-1-naphthol in einer Mischung von 3 l Eisessig und 300 ml Chloroform unter Zugabe von 180 g (1,33 mol) Natriumacetat-trihydrat. (Bei der Herstellung der Lösung des Naphtholethers ist darauf zu achten, daß nach Eintragen desselben in Eisessig-Chloroform unter Zugabe von Natriumacetat nach einem Temperaturanstieg auf ca. 45°C wieder auf 20°C abgekühlt wird.) Nach 3 Stunden Rühren im Eisbad wird das erzeugte Kristallisat abgesaugt, der Rückstand dreimal mit je 500 ml Wasser gewaschen und im Trockenschrank bei 40°C getrocknet. Das Rohprodukt - 295,5 g hellbraune Kristalle - werden chromatographisch gereinigt. Die Azoverbindung wird in 1 l Toluol/Methylenchlorid 2:5 gelöst auf eine Kieselgel 60 (Merck)-säule mit innerem Durchmesser 11,5 cm, Füllhöhe 1,2 m aufgetragen und mit Toluol/Methylenchlorid 2:5 eluiert. Man schneidet Fraktionen von ca. 70 ml. Die Fraktionen 57-173 werden vereinigt und eingeengt. Man erhält 134,2 g braune Kristalle. Diese werden in 480 ml Toluol bei 80°C gelöst, auf 65°C gekühlt und unter kräftigem Rühren mit 800 ml Isohexan versetzt. Man läßt unter Rühren auf 20°C abkühlen, stellt über Nacht in den Kühlschrank, saugt die gebildeten Kristalle ab und wäscht den Filterköcher mit 2 mal 300 ml eiskaltem Toluol/Isohexan 1:1,3 und anschließend mit 300 ml Isohexan. Anschließend trocknet man im Trockenschrank bei 40°C über Diphosphorpentoxid bis zur Gewichtskonstanz. Man erhält 119,9 g (55,5 % der Theorie) Azoverbindung, hellbraune Kristalle, Fp. 102-103°C

Analog Beispiel 5 a) - c) kann auch die folgende Substanz hergestellt werden:

d) 4-[(2-Brom-4-nitro-6-trifluormethylphenyl)azo]-2-octadecyloxy-1-naphthol, Fp. 84°C aus 2-Brom-4-nitro-6-trifluormethylanilin (M. Hauptschein et al., J. Amer. Chem. Soc. 76, 1051 (1954))

Beispiel 6

2,4,6,8-Tetranitro-5-octadecyloxy-1-naphthol

a) 5-Octadecyloxy-1-naphthol

In einem 2 l Dreihalskolben mit Claisenaufsatz, Thermometer, Calciumchloridrohr und Tropfrichter suspendiert man 40 g (0,25 mol) 1,5-Dihydroxynaphthalin (Janssen 99 %) in 400 ml frisch destilliertem Dimethylformamid und gibt unter Rühren 6 g (0,25 mol) 97 % Natriumhydrid in kleinen Portionen innerhalb 40 Minuten zu. Dabei tritt neben Wasserstoffentwicklung und Temperaturanstieg auf 36°C Lösung unter Blaufärbung ein. Man rührt noch 30 Minuten nach und tropft in die noch 35°C warme Lösung 83,3 g (0,25 mol) 96% 1-Octadecylbromid innerhalb 10 Minuten zu. Danach rührt man 24 Stunden bei Raumtemperatur nach. Das angefallene Rohprodukt wird scharf abgesaugt und der Rückstand 15 Minuten mit 600 ml Wasser angerührt. Diese Prozedur wird nochmals wiederholt und der Filterrückstand solange mit Wasser (ca. 800 ml) gewaschen, bis das Filtrat farblos ist.

Danach trocknet man den Filterkuchen bei 40°C im Trockenschrank über Diphosphorpentoxid. Man erhält 98,6 g hellbeige Kristalle vom Schmelzpunkt 76-78°C.

Zur Weiterreinigung rührt man das Produkt dreimal mit je 750 ml Essigsäureethylester an, filtriert die ungelösten Anteile (40,8 g) hellbeige Kristalle ab, behandelt die Mutterlauge zweimal mit Kohle und engt im Vakuum ein. Man erhält 53,2 g (51,9 % der Theorie) beigefarbene Kristalle vom Schmelzpunkt 90-92°C. Dieses Produkt wird direkt für die Herstellung der tetranitrierten Verbindung verwendet. DC Kieselgel 60 (Merck), Laufmittel: Toluol/Methanol 50:1; $R_f$ = 0,36.

b) 2,4,6,8-Tetranitro-5-octadecyloxy-1-naphthol

In einem 2 l Dreihalskolben mit großflächigem Rührer und Thermometer gibt man 1,2 l konz. Schwefelsäure, erwärmt auf 40°C und gibt 49,52 g (0,12 mol) 5-Octadecyloxy-1-naphthol möglichst rasch unter kräftigem Rühren zu. Nach 5-10 Minuten entsteht unter 2-3°C Temperaturanstieg ein dicker Kristallbrei. Man rührt dann 20 Minuten ohne Heizung nach, kühlt dann auf ca. 0°C ab und tropft die Nitriersäure (hergestellt aus 34,9 ml Salpetersäure 65prozentig; unter Rühren und Kühlen bei ca. 10-20°C zu 70 ml konz. $H_2SO_4$ in ca. 15 Minuten eingetragen) bei 0-5°C innerhalb 30 Minuten zu. Dabei färbt sich die Reaktionsmischung graubraun bis rotbraun. Nach vierstündigem Nachrühren bei 5-10°C wird auf ca. 5 kg Eis gegossen und das Rohprodukt 3 mal mit 2 1 Essigsäurethylester extrahiert. Danach werden die Essigsäureethylesterphasen vereinigt, zweimal mit je 1 l Wasser gewaschen, der Essigsäureethylester über Natriumsulfat getrocknet, abgesaugt und eingeengt. Man erhält ca. 80 g dunkelbraunen harzigen Rückstand. Dieser wird säulenchromatographisch gereinigt. Man verwendet eine Säule von 7,5 cm innerem Durchmesser, Füllhöhe ca. 110 cm, Füllmittel Kieselgel 60 (Merck), Laufmittel: Toluol/Aceton 5:2. Haupfraktion $R_f$ = 0,24.

Diese Rohsubstanz wird nochmals mit ca. 400 ml Laufmittel angerührt, falls nicht alles gelöst, abgesaugt (Rückstand kann die Säule verstopfen) und das Filtrat auf die Säule gegeben und fraktionsweise eluiert. Man schneidet Fraktionen zu je 80 ml. Vorlauf (farbloses Eluat) ca. 2 l. Die Substanz enthaltenden Fraktionen (30-140) werden eingeengt. Man erhält 21 g rotbraunen zähen Brei, der nach längerem Stehen durchkristallisiert. Man löst dieses Produkt in 42 ml Aceton und fällt das Endprodukt durch langsame Zugabe der 5fachen Menge Isohexan bei Raumtemperatur. Nach fünfstündigem Rühren saugt man ab, wäscht den Filterkuchen mit Isohexan und trocknet im Vakuum über Diphosphorpentoxid und Molekularsieb bei Raumtemperatur. Man erhält 14,9 g (21 % der Theorie) des gewünschten Tetranitrooctadecyloxynaphthols, Fp. 236-238°C (Zers.). DC: Kieselgel 60 (Merck), Laufmittel: Methylenchlorid/Methanol 8:1; $R_f$ = 0,27.

Beispiel 7

3-Pentadecyl-2,4,6-trinitrophenol

In einem 500 ml Erlenmeyerkolben werden 67.5 g (0,2 mol) Pentadecylphenol 90 % unter Rühren in 100 ml konz. Schwefelsäure eingetragen, auf 90°C erwärmt, wobei eine schwarzbraune, hochviskose, schwer rührbare Masse entsteht, die eine Stunde bei 90°C gehalten wird. In einem separaten 1 l Dreihalskolben werden 70 ml (ca. 1 mol) 65proz. Salpetersäure mit Hilfe eines Eisbades auf 10°C gekühlt. Das hochviskose zuvor erhaltene Sulfonierungsprodukt wird während ca. 2 Stunden in kleinen Portionen (wobei die viskose Masse mit einem Fön flüssig gehalten wird) unter Eisbadkühlung so zu der Salpetersäure gegeben, daß die Temperatur von 25°C nicht überschritten wird und erhält dabei eine beigefarbene schwer rührbare Masse, die man eine Stunde bei Raumtemperatur weiterrührt. Danach gießt man

auf 500 g Eis, wobei sich ein feiner Niederschlag bildet. Das so erhaltene Rohprodukt läßt sich nur schwer absaugen, weshalb man das Ganze am besten an der Zentrifuge schleudert. Nach Abdekantieren der überstehenden Flüssigkeit wird diese Prozedur zur Entfernung von anhaftender Säure zweimal nach jeweiliger Zugabe von Wasser wiederholt, der resultierende Niederschlag mit 500 ml Ethanol in einen Kolben gespült, durch Erwärmen auf 50°C (im Wasserbad) in Lösung und durch Einstellen in ein Eisbad zur Kristallisation gebracht. Nach scharfem Absaugen des Produktes erhält man 37,5 g (42,6 % der Theorie) schwach ethanolfeuchtes, beigefarbenes 3-Pentadecyl-2,4,6-trinitrophenol, Schmelzpunkt 53-56°C, DC: Kieselgel 60, Laufmittel: Ethylacetat/Methanol/Eisessig 90:5:5, $R_f = 0,8$.

Nach Trocknen der Substanz über Diphosphorpentoxid erhält man 35,75 g (40,1 % der Theorie) 3-Pentadecyl-2,4,6-trinitrophenol, Fp. 59-61°C.

Beispiel 8

[(2,4-Dinitrophenyl)hydrazono]propandinitril

Man löst 2,1 g (0.03 mol) Natriumnitrit in 30 ml konz. Schwefelsäure. Dabei steigt die Temperatur auf 50°C an. Man kühlt auf 20°C ab, tropft 20 ml Eisessig bei 15 - 20°C zu und gibt 5,5 g (0,03 mol) 2,4-Dinitroanilin (NL-A-6411189) portionsweise zu und rührt noch eine Stunde bei 20°C nach.

Man löst 1,98 g Malondinitril in 75 ml Ethanol, gibt eine Lösung von 74 g Natriumacetat-trihydrat in 35 ml Wasser zu und tropft unter Rühren bei 19°C die vorstehend hergestellte Diazoniumsalzlösung zu.

Chromatographische Aufreinigung der Reaktionsmischung nach 1 Stunde an Kieselgel 60 (Merck) mit Methylenchlorid/Methanol = 98:2 ergibt das Produkt mit Rf = 0,28.

**Patentansprüche**

**1.** Testträger zur Bestimmung von Ionen enthaltend

eine Testschicht, die eine flüssigkeitsfeste organische Phase aufweist, die ein hydrophobes Polymer in homogener Mischung mit einer schwer flüchtigen hydrophoben organischen Flüssigkeit enthält

und einen Ionophor

sowie eine Substanz, die in Anwesenheit des zu bestimmenden Ions ihre Farbe ändert, dadurch gekennzeichnet, daß die Testschicht Partikel mit einer Ölzahl von 80 - 200 enthält, und der Ionophor in der hydrophoben organischen Phase homogen verteilt ist.

**2.** Testträger gemäß Anspruch 1, dadurch gekennzeichnet, daß das hydrophobe Polymer ein Copolymer des Vinylacetats ist.

**3.** Testträger gemäß einem der Ansprüche 1 oder 2, dadurch gekennzeichnet, daß als Partikel solche eingesetzt werden, die Hohlräume aufweisen.

**4.** Testträger gemäß einem der Ansprüche 1 - 3, dadurch gekennzeichnet, daß das Gewichtsverhältnis von hydrophobem Polymer zu Partikel in der Testschicht etwa 5:1 bis etwa 1:10, insbesondere etwa 1:1 bis etwa 1:3 beträgt.

**5.** Testträger gemäß einem der Ansprüche 1 bis 4, dadurch gekennzeichnet, daß die schwer flüchtige, hydrophobe organische Flüssigkeit ausgewählt ist aus der Gruppe der Sebacinsäure-, Phthalsäure-, Acrylsäure-, Phosphorsäureester und Silicone.

**6.** Testträger gemäß einem der Ansprüche 1 - 5, dadurch gekennzeichnet, daß das Gewichtsverhältnis von hydrophobem Polymer zu schwer flüchtiger, hydrophober organischer Flüssigkeit in der Testschicht etwa 5:1 bis etwa 1:5, insbesondere etwa 2:1 bis etwa 1:2 beträgt.

**7.** Testträger gemäß einem der Ansprüche 1 bis 6, dadurch gekennzeichnet, daß als farbändernde Substanz eine Verbindung der allgemeinen Formel I

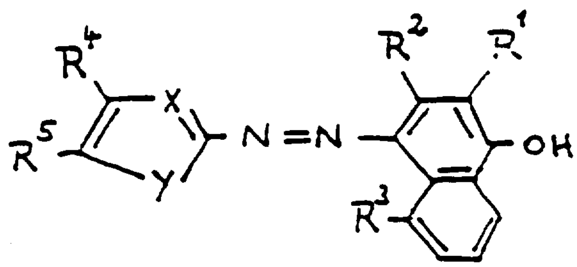

(I)

in der

| | |
|---|---|
| $R^1$, $R^2$, $R^3$ | gleich oder verschieden sind und Wasserstoff, eine Alkyl- oder Alkoxygruppe, darstellen, wobei mindestens einer der Reste ein ($C_8$-$C_{30}$)-Alkyl- oder Alkoxyrest ist, |
| $R^4$ | Wasserstoff oder eine Alkylgruppe, |
| $R^5$ | eine Nitrogruppe, eine durch Halogen substituierte Alkylgruppe, eine Cyanogruppe, eine Sulfonamidgruppe oder eine Alkylsulfonylgruppe, |
| X | Stickstoff oder den Rest $CR^6$ und |
| Y | Schwefel oder den Rest $CR^7 = CR^8$ |

wobei $R^6$, $R^7$, $R^8$ gleich oder verschieden sind und
Wasserstoff, Halogen, eine Nitrogruppe, eine Cyanogruppe, eine Alkyl- oder durch Halogen substituierte Alkylgruppe oder eine Alkylsulfonylgruppe
bedeuten, eingesetzt wird.

**8.** Testträger gemäß einem der Ansprüche 1 bis 7, dadurch gekennzeichnet, daß die Testschicht eine Substanz aus der Gruppe der höher nitrierten Alkylphenole, höher nitrierten Alkoxynaphthole und Carbonyldicyanidphenylhydrazone enthält.

**9.** Testträger gemäß einem der Ansprüche 1 bis 8, dadurch gekennzeichnet, daß die Testschicht zusätzlich eine Puffersubstanz enthält.

**10.** Testträger gemäß einem der Ansprüche 1 bis 9, dadurch gekennzeichnet, daß farbändernde Substanz und flüssigkeitsfeste organische Phase in zwei Testschichten enthalten sind, die miteinander in einen Flüssigkeitsaustausch ermöglichenden direkten oder indirekten Kontakt gebracht werden können.

**Claims**

**1.** Test carrier for the determination of ions, containing a test layer which has a liquid-stable organic phase which contains a hydrophobic polymer in homogeneous mixture with a non-volatile hydrophobic organic liquid and an ionophore, as well as a substance which changes its colour in the presence of the ion to be determined, characterised in that the test layer contains particles with an oil number of 80 -200 and the ionophore is homogeneously distributed in the hydrophobic organic phase.

**2.** Test carrier according to claim 1, characterised in that the hydrophobic polymer is a co-polymer of vinyl acetate.

**3.** Test carrier according to one of claims 1 or 2, characterised in that, as particles, those are used which have hollow spaces.

**4.** Test carrier according to one of claims 1 - 3, characterised in that the weight ratio of hydrophobic polymer to particles in the test layer amounts to about 5:1 to about 1:10, especially about 1:1 to about 1:3.

**5.** Test carrier according to one of claims 1 to 4, characterised in that the non-volatile hydrophobic organic liquid is selected from the group of sebacic acid esters, phthalic acid esters, acrylic acid esters, phosphoric acid esters and silicones.

**6.** Test carrier according to one of claims 1- 5, characterised in that the weight ratio of hydrophobic polymer to non-volatile hydrophobic organic liquid in the test layer amounts to about 5:1 to about 1:5, especially about 2:1 to about 1:2.

**7.** Test carrier according to one of claims 1 to 6, characterised in that, as colour-changing substance, there is used a compound of the general formula I

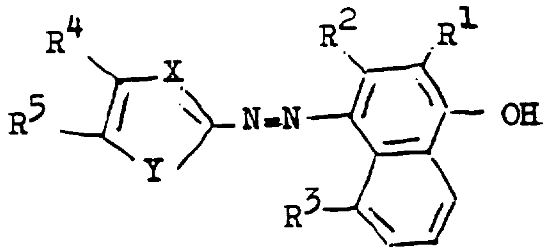

(I)

in which $R^1$, $R^2$, $R^3$ are the same or different and represent hydrogen, an alkyl or alkoxy group, whereby at least one of the radicals is a ($C_8$-$C_{30}$)-alkyl or -alkoxy radical, $R^4$ signifies hydrogen or an alkyl group, $R^5$ a nitro group, an alkyl group substituted by halogen, a cyano group, a sulphonamide group or an alkylsulphonyl group, X nitrogen or the radical $CR^6$ and Y sulphur or the radical $CR^7$=$CR^8$, whereby $R^6$, $R^7$, $R^8$ are the same or different and signify hydrogen, halogen, a nitro group, a cyano group, an alkyl group or an alkyl group substituted by halogen or an alkylsulphonyl group.

**8.** Test carrier according to one of claims 1 to 7, characterised in that the test layer contains a substance from the group of the highly nitrated alkylphenols, highly nitrated alkoxynaphthols and carbonyldicyanide phenyl hydrazones.

**9.** Test carrier according to one of claims 1 to 8, characterised in that the test layer additionally contains a buffer substance.

**10.** Test carrier according to one of claims 1 to 9, characterised in that colour-changing substance and liquid-stable organic phase are contained in two test layers which can be brought into direct or indirect contact with one another making possible a liquid exchange.

**Revendications**

**1.** Support de test pour la détermination d'ions, contenant

une couche de test qui présente une phase organique résistant aux liquides, qui contient un polymère hydrophobe en mélange homogène avec un liquide organique hydrophobe peu volatil

et un agent ionophore

ainsi qu'une substance qui, en présence de l'ion à déterminer, subit une variation de couleur, caractérisé par le fait que la couche de test contient des particules ayant un indice d'huile de 80-200, et que l'agent ionophore est réparti de façon homogène dans la phase organique hydrophobe.

**2.** Support de test selon la revendication 1, caractérisé par le fait que le polymère hydrophobe est un copolymère d'acétate de vinyle.

**3.** Support de test selon l'une des revendications 1 ou 2, caractérisé par le fait qu'en tant que particules, on utilise des particules présentant des volumes vides.

**4.** Support de test selon l'une quelconque des revendications 1 à 3, caractérisé par le fait que le rapport en poids du polymère hydrophobe aux particules dans la couche de test est d'environ 5 :1 à environ 1 :10, en particulier d'environ 1 : 1 à environ 1 : 3.

**5.** Support de test selon l'une quelconque des revendications 1 à 4, caractérisé par le fait que le liquide organique hydrophobe peu volatil est choisi dans le groupe formé par les esters de l'acide sébacique, de l'acide phtalique, de l'acide acrylique, de l'acide phosphorique et le silicone.

**6.** Support de test selon l'une quelconque des revendications 1 à 5, caractérisé par le fait que le rapport en poids du polymère hydrophobe au liquide organique hydrophobe peu volatil dans la couche de test est d'environ 5 : 1 à

environ 1 : 5, en particulier d'environ 2 : 1 à environ 1 : 2.

7. Support de test selon l'une quelconque des revendications 1 à 6, caractérisé par le fait qu'en tant que substance subissant une variation de couleur, on utilise un composé de formule générale I

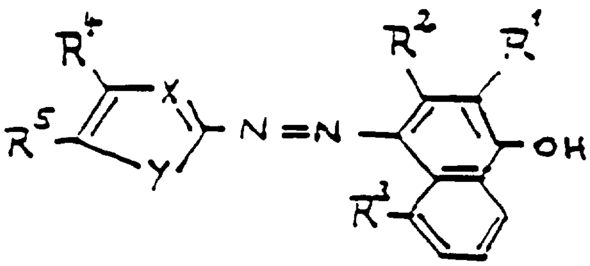

(I)

dans laquelle

| | |
|---|---|
| $R^1$, $R^2$, $R^3$ | sont identiques ou différents et représentent un atome d'hydrogène, un groupe alkyle ou alcoxy, au moins l'un des restes étant un reste alkyle en $C_8$-$C_{30}$ ou alcoxy en $C_8$-$C_{30}$, |
| $R^4$ | représente un atome d'hydrogène ou un groupe alkyle, |
| $R^5$ | représente un groupe nitro, un groupe alkyle halogéné, un groupe cyano, un groupe sulfonarnido ou un groupe alkylsulfonyle, |
| X | représente un atome d'azote ou le reste $CR^6$ et |
| Y | représente un atome de soufre ou le reste $CR^7 = CR^8$, |

où les restes $R^6$, $R^7$, $R^8$ sont identiques ou différents et représentent un atome d'hydrogène, un atome d'halogène, un groupe nitro, un groupe cyano, un groupe alkyle ou un groupe alkyle halogéné ou un groupe alkylsulfonyle.

8. Support de test selon l'une quelconque des revendications 1 à 7, caractérisé par le fait que la couche de test comprend une substance appartenant au groupe constitué par les alkylphénols nitrés plusieurs fois, les alcoxynaphtols nitrés plusieurs fois et la carbonyldicyanidephénylhydrazone (phénylhydrazonopropanedinitrile).

9. Support de test selon l'une quelconque des revendications 1 à 8, caractérisé par le fait que la couche de test contient en outre une substance tampon.

10. Support de test selon l'une quelconque des revendications 1 à 9, caractérisé par le fait que la substance subissant une variation de couleur et la phase organique résistant aux liquides sont contenues dans deux couches de test qui peuvent être amenées en un contact mutuel direct ou indirect, permettant un échange de liquides.

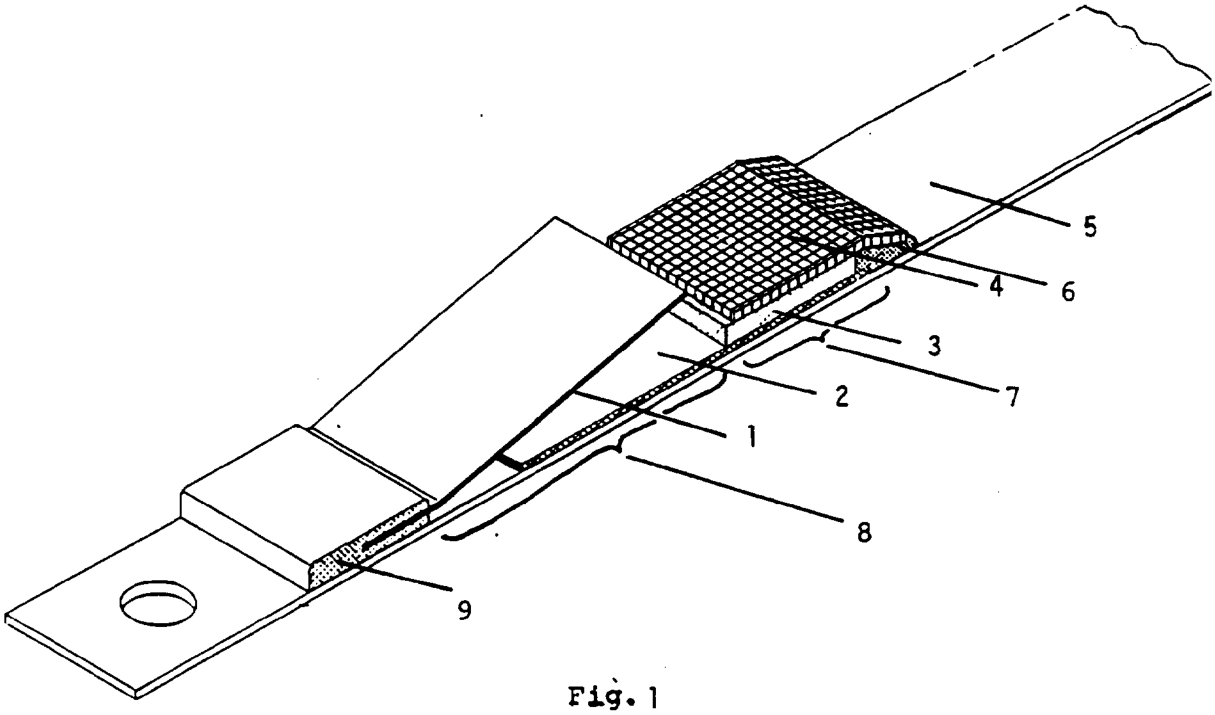


Fig. 1

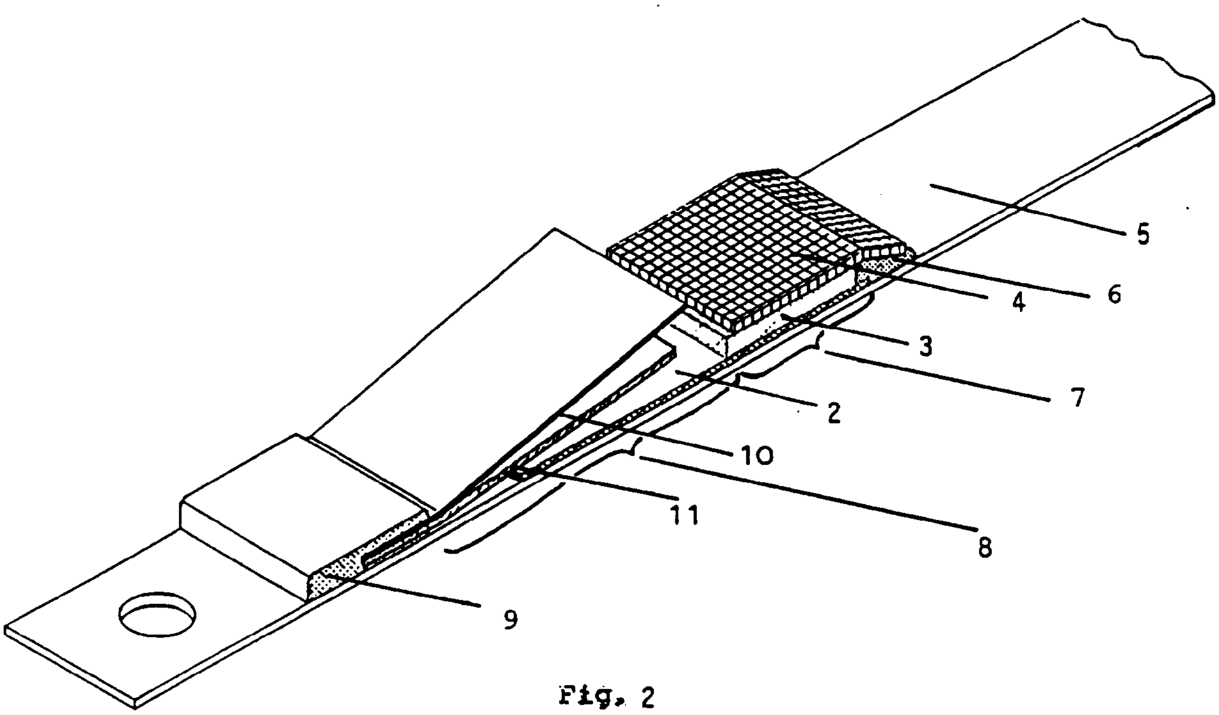


Fig. 2